# EUROPÄISCHE PATENTANMELDUNG

(11) **EP 1 946 657 A2**
(43) Veröffentlichungstag der Anmeldung: **23.07.2008**
(21) Anmeldenummer: 08000533.3
(22) Anmeldetag: 12.01.2008
(51) Int. Cl.: A23L 2/02, A23F 3/14, A23L 1/304, A23L 1/30, A23L 1/302

(54) **Verfahren zum Unterstützen und Ergänzen der Wirkung eines Saunabades**

(30) Priorität: 22.01.2007 DE 102007003194
(71) Anmelder: Morlo-Roth, Claus-Dieter, 77704 Oberkirch (DE)
(72) Erfinder: Morlo-Roth, Claus-Dieter, 77704 Oberkirch (DE)
(74) Vertreter: Bernhardt, Reinold

(57) **Zusammenfassung**

Zum Unterstützen und Ergänzen der Wirkung eines Saunabades soll vor dem ersten Saunagang und zwischen den Saunagängen ein entschlackend wirksames Getränk verabreicht werden.
Das erste Getränk ist als Heißgetränk vorgesehen. Dadurch wird eine den Saunagang vorbereitende innere Vorwärmung erzeugt und das Ingangkommen des Schwitzens beschleunigt.
Die Getränke zwischen den Saunagängen sollen entspannend wirksam sein. Die Entspannung ist bekanntermaßen eine grundlegende Komponente einer erfolgreichen Saunakur.
Nach dem letzten Saunagang soll ein mit Mineralstoffen, vorzugsweise ferner mit Vitaminen, angereichertes Getränk gegeben werden, um die beim Saunabad ungewollt ausgeschiedenen Stoffe im Körper möglichst wieder zu ersetzen.

Bevorzugt als die genannten Getränke sind Teemischungen.

## Beschreibung

Die Erfindung betrifft ein Verfahren zum Unterstützen und Ergänzen der Wirkung eines Saunabades.

Die reinigende, entschlackende Wirkung des Saunabadens ist bekannt.

Der Erfindung liegt die Aufgabe zugrunde, sie zu fördern und zu ergänzen.

Gemäß der Erfindung wird dieser Zweck erfüllt, indem vor dem ersten und/oder mindestens einem weiteren Saunagang ein entschlackend wirksames Getränk verabreicht wird.
Das Getränk ist vorgesehen, im Sinne einer Durchspülung und Anregung sowie Förderung der körpereigenen Mechanismen zum Schadstoffabbau, insbesondere im Zusammenspiel mit der thermisch angeregten Entschlackung und Reinigung, d.h. auch Entgiftung, durch die Schwitzkur in der Sauna, zu wirken.

Vorzugsweise wird vor dem ersten Saunagang das Getränk als Heißgetränk verabreicht.
Dadurch wird eine den Saunagang vorbereitende innere Vorwärmung erzeugt und das Ingangkommen des Schwitzens beschleunigt. Es erfolgt damit auch eine psychische Programmierung auf die Saunakur.

Nach einer Weiterbildung der Erfindung wird vor mindestens einem weiteren Saunagang ein entspannend wirksames Getränk verabreicht, das vorzugsweise zugleich ein Getränk der vorgenannten Art ist.

Die Entspannung ist bekanntermaßen eine grundlegende Komponente einer erfolgreichen Saunakur, die auch regenerativen und erholsamen Charakter haben soll.

Das entspannend wirksame, in der Regel einen beruhigenden Wirkstoff enthaltende, Getränk kann je nach persönlicher Konstitution und Intention kalt, jedoch nicht zu kalt, oder heiß gegeben werden, um zu erfrischen oder den Nachschwitzeffekt zu verstärken.

Schließlich wird vorgeschlagen, die Verfahrensweise zu einer vollständigen kombinierten Trink- und Saunakur auszugestalten, indem nach dem letzten Saunagang ein mit Mineralstoffen, vorzugsweise ferner mit Vitaminen, angereichertes Getränk gegeben wird, um die beim Saunabad ungewollt ausgeschiedenen Stoffe im Körper möglichst wieder zu ersetzen.

Die Erfindung stellt ein gesundheitsförderndes Ergänzungsangebot im Rahmen der Wellness- und Healthness-Programme gewerblicher Saunabadbetreiber zur Verfügung.
Für den privaten Bereich können käufliche Trinkeinheiten von z.B. 0,3 1 mit der erklärten Bestimmung zum Einnehmen vor dem ersten bzw. einem weiteren bzw. nach dem letzten Saunagang oder entsprechende Mengen von Ausgangssubstanzen zur Bereitung der Getränke angeboten werden.

Es sei festgehalten, dass die in Betracht stehenden Getränke die Wirkstoffe nur in einer milden Dosierung enthalten sollen und nicht in einem medizinisch therapeutischen Sinne vorgesehen sind.

Besonders, wenn auch nicht allein, geeignet sind Teemischungen.

Im folgenden sind als Ausführungsbeispiele der Erfindung zwei mit Saunabaden zu kombinierende Trinkkuren angegeben.
I.

### 1) Heißgetränk vor dem ersten Saunagang:

Teemischung aus 17% Apfelstücken (Fruchtbasis des Tees), 17% Pfefferminze (Aromaträger, erfrischend, gallenflussfördernd, tonisierend), 15% Hibiskusblüten (Aromaträger, harn- und galletreibend), 10% Ananasstücke (immunmodulierend, blutdruckregulierend), 9% Heidelbeeren (Anregung des Stoffwechsels), 7% Hagebuttenschalen (Aromaträger, Stärkung des Immunsystems), 5% Birkenblätter (blutreinigend, harntreibend, entschlackungsfördernd), 5% Zitrusschalen (Aromaträger, aufbauend), 5% Süßholz (immunmodulierend, blutreinigend, anregend und damit entschlackungsfördernd), 5% Brennnesselblätter (wassereinlagerungsregulierend, blutreinigend, blutbildend, die Nierentätigkeit anregend), 5% Holunder (schweißtreibend, anregend).

Die Mengen der Zutaten sind, wie auch bei den folgenden Tees, unter dem Aspekt des geschmacklichen sowie wirkseitigen Zusammenspiels im Sinne eines gesundheitsfördernden Wellness-Tees bemessen.

### 2) Heiß- oder Kaltgetränk vor weiteren Saunagängen:

Teemischungen aus 17% Apfelstücken, 15% Hibiskusblüten, 10% Ananasstücken, 10% Heidelbeeren, 10% Kamille (beruhigend, die Entschlackung und die Entspannung fördernd), 8% Pfefferminze, 7% Melisse (beruhigend, krampflösend), 5% Baldrian (beruhigend), 5% Birkenblätter, 5% Süßholz, 4% Holunderbeeren, 4% Zitrusschalen.

Die Rezeptur ist verstärkt auf den Aspekt der Entspannung ausgerichtet.

### 3) Heiß- oder Kaltgetränk nach dem Saunabaden:

Teemischung aus 22,5% Apfelstücken (hier: Inhaltsstoff Biotin), 12,5 % Hagebuttenschalen (Vitamin C), 9,5% Pfirsichstücken (Vitamin E, Niacin), 5% Sanddornbeeren (Vitamin C), 5% Aroniakompaktat (aus Aroniasaft; Vitamine E,K,B2,B9 und P, Eisen, Jod, Provitamin A), 4% rote Beete (Kalium, Magnesium, Eisen, Fluor), 4% Karottenstücke (Provitamin A, Vitamine B1 und B2, Kalium, Eisen), 4% Süßholz, 4% Pflaumenstücke (Kalium), 4% Sauerkirschen (Vitamin E, Kupfer, Kalium), Cranberries (Vitamin C, Phosphor, Zink), 4% Macadamianüsse (Vitamin B1, Magnesium, Kupfer), 3% Zitrusschalen (Provitamin A, Vitamin C), 2,5% Mandeln (Magnesium), 2% Preiselbeeren (Vitamin C).
Calcium und Natrium werden vorteilhafterweise dadurch zugeführt, dass der Tee mit einem kohlensäurefreien Mineralwasser aufgebrüht wird.

### 1) Heißgetränk vor dem ersten Saunagang:

Teemischung aus 36% Rooibuschtee (Basistee; beruhigend, entspannend), 14% Apfelstücken, 10% Ananasstücken, 7% Ingwer (darmfunktionssteigernd, kreislaufanregend, temperaturerhöhend, schweißtreibend), 6% Zitrusschalen, 6% Artischockenblätter (gallenbildungs- und gallenflussfördernd), 5% Löwenzahn (blutreinigend, blutbildend, harntreibend, tonisierend, mineralreich), 5% Ringelblumenblüten (anregend, reinigend, krampflösend, schweißtreibend), 5% Süßholz, 3% Vanille (Aromaträger; anregend), 3% Kardamom (anregend, krampflösend, gallensekretionsfördernd).

### 2) Heiß- oder Kaltgetränk vor weiteren Saunagängen:

Teemischung aus 35% Rooibuschtee, 14% Apfelstücken, 10% Ananasstücken, 5% Zitrusschalen, 5% Hopfen (beruhigend), 5% Johanniskraut (beruhigend), 5% Lavendel (beruhigend), 5% Löwenzahn, 5% Ringelblumenblüten, 5% Süßholz, 3% Vanille, 3% Kardamom.

### 3) Getränk nach dem Saunabaden:

Teemischung aus 62% Grüntee Sencha (Vitamin C), 5% Aroniakompaktat, 5% Sanddornbeeren, 5% Zitrusschalen, 5% Karottenstücken, 5% Kürbiskernen (Vitamin E), 3% Lemongras (Vitamin C), 2,5% Orangenstücken (Provitamin A, Vitamin C), 2,5% Macadamianüssen, 2% Lemon Myrte, 2% Rote Beete, 1% Spirulina Granulat (Jod).

Dieser Tee ist wegen der Bitterstoffe im Grüntee weniger als Kaltgetränk geeignet.

## Patentansprüche

1. Verfahren zum Unterstützen und Ergänzen der Wirkung eines Saunabades,
**dadurch gekennzeichnet,**
**dass** vor dem ersten und/oder mindestens einem weiteren Saunagang ein entschlackend wirksames Getränk verabreicht wird.

2. Verfahren nach Anspruch 1,
**dadurch gekennzeichnet,**
**dass** vor dem ersten Saunagang ein warmes Getränk der genannten Art verabreicht wird.

3. Verfahren nach Anspruch 1 oder 2,
**dadurch gekennzeichnet,**
**dass** vor mindestens einem weiteren Saunagang ein entspannend wirksames Getränk, vorzugsweise zugleich ein Getränk der genannten Art, verabreicht wird.

4. Verfahren nach einem der Ansprüche 1 bis 3,
**dadurch gekennzeichnet,**
**dass** nach dem letzten Saunagang ein mit Mineralstoffen, vorzugsweise ferner mit Vitaminen, angereichertes Getränk verabreicht wird.

5. Verfahren nach einem der Ansprüche 1 bis 4,
**dadurch gekennzeichnet,**
**dass** das Getränk eine Teemischung ist.

6. Entschlackend und/oder entspannend wirksames Getränk zum Einnehmen vor einem Saunagang oder Ausgangssubstanz zur Bereitung eines solchen Getränks.

7. Mit Mineralstoffen, vorzugsweise ferner mit Vitaminen, angereichertes Getränk zum Einnehmen nach einem Saunabad oder Ausgangssubstanz zur Bereitung eines solchen Getränks.
